# EUROPÄISCHE PATENTSCHRIFT

(11) **EP 3 556 413 B1**
(45) Veröffentlichungstag und Bekanntmachung des Hinweises auf die Patenterteilung: **13.07.2022**
(21) Anmeldenummer: 19000224.6
(22) Anmeldetag: 26.09.2014
(51) Int. Cl.: A61M 13/00, A61B 90/00, A61M 16/16, A61M 16/20

(54) **DRUCKERHALTENDE RAUCHGASABSAUGUNG IN EINEM INSUFFLATOR**
PRESSURE-MAINTAINING SMOKE EXTRACTION IN AN INSUFFLATOR
ASPIRATION D'EFFLUENTS GAZEUX À MAINTIEN DE LA PRESSION DANS UN INSUFFLATEUR

(30) Priorität: 27.09.2013 DE 102013016063
(43) Veröffentlichungstag der Anmeldung: 23.10.2019
(62) Teilanmeldung aus: 14795539.7
(73) Patentinhaber: W.O.M. World of Medicine GmbH, 10587 Berlin (DE)
(72) Erfinder: Köth, Yves, 12683 Berlin (DE); Stegemann, Sebastian, 12105 Berlin (DE)
(74) Vertreter: Jungblut & Seuss

(56) Entgegenhaltungen:
- WO-A1-2004/009167
- WO-A1-2011/041387
- WO-A1-2012/012379
- DE-A1- 4 219 859
- DE-C1- 3 922 746
- US-A- 4 735 603
- US-A- 4 836 187
- US-A- 4 971 034

## Beschreibung

Die vorliegende Erfindung betrifft einen Insufflator mit einer Rauchgasabsaugung welcher so ausgestaltet ist, dass der abdominelle Druck während der Absaugung weitestgehend konstant bleibt. Die Anmeldung ist eine Teilanmeldung der früheren europäischen Patentanmeldung EP14795539.7 (Anmeldetag 26.09.2014). Die Priorität der deutschen Anmeldung DE102013016063.4 (Anmeldetag 27.9.2013) wird in Anspruch genommen.

### Hintergrund und Stand der Technik

Im Bereich der minimal-invasiven Chirurgie, zum Beispiel der diagnostischen oder therapeutischen Laparoskopie wird im Abdomen mittels eingeleiteter Gase (z. B. medizinisches Kohlendioxid (CO₂)) ein Überdruck erzeugt, welcher das Abdomen aufdehnt, damit ausreichend Platz für die visuelle Inspektion bzw. den therapeutischen Eingriff besteht. Hierzu werden sogenannte Insufflatoren verwendet, welche mittels eines geregelten Gasflusses einen kontrollierten Druck im Abdomen aufbauen. Über eine Reihe von Sicherheitsvorrichtungen wird sichergestellt, dass sich der abdominelle Druck in Grenzen hält, so dass kein Gewebe geschädigt wird.

Im Rahmen der minimal-invasiven Chirurgie werden häufig elektrochirurgische Geräte oder auch Laser verwendet, deren Anwendung Rauch im Abdomen erzeugt. Dieser Rauch behindert nicht nur die Sicht, sondern enthält auch Schadstoffe (in Form von Gasen, Tröpfchen und/oder Partikel), so dass dieser aus dem Abdomen entfernt werden muss. Eine Lösung dieses Problems besteht darin, einen einfachen Auslass zu schaffen, über den die Rauchgase den Körper verlassen. Eine Realisierungsmöglichkeit hierfür wird in der US 6592543 gezeigt. Die Absaugung wird dabei durch den Operateur manuell durch das öffnen des Ventils am Trokar gesteuert. Allerdings werden durch heutige Systeme häufig größere Rauchmengen erzeugt, als zum Anmeldezeitpunkt der US 6592543, so dass die dort vorgestellte Lösung heute nicht mehr anwendbar ist.

Eine Weiterentwicklung derart passiver Systeme besteht durch die Bereitstellung einer Absaugvorrichtung, die das Gas im Abdomen inklusive der Rauchpartikel aktiv absaugen kann. Ein derartiges System wird beispielsweise in der US 5199944 beschrieben. Dabei besteht das Problem, dass bei zu starker Absaugung der Rauchgase der Druck im Abdomen sinkt, so dass das Abdomen in sich zusammenfallen kann. Der behandelnde Arzt muss dann mit der Weiterführung der Operation solange warten, bis sich im Abdomen wieder ausreichend Druck aufgebaut hat. Es ist ohne weiteres einsichtig, dass diese Vorgehensweise enorme Nachteile mit sich bringt. Anderseits ist bei zu schwacher Absaugung diese nicht effektiv genug, so dass der Operateur durch die schlechte Sicht behindert wird und die Operation verlängert wird. Eine Alternative zu dieser Vorgehensweise besteht darin, das abgesaugte Gas dem Patienten wieder zuzuführen (Zirkulation). Ein derartiges System wird in dem Patent US 4735603 beschrieben. Auch in dieser Ausführungsform besteht das Problem, das bei zu hoher Absaugrate der abdominelle Druck sinkt.

Bei einer hohen Absaugleistung steigt der Systemdruck auf der Zuführleitung stark an. Je nachdem, wie eng das angeschlossene Instrument ist, kann der Druck stärker oder schwächer ansteigen. In jedem Fall wird ab einer bestimmten Druckgrenze das Sicherheitsventil innerhalb der Zuführleitung öffnen und schon damit zu einem Druckabfall führen. Weiterhin kann der Insufflator kein zusätzliches Kohlendioxid mehr zuführen, um wahrscheinliche vorhandene Leckagen auszugleichen.

Sowohl bei einer Absaugung mit als auch ohne Zirkulation ergibt sich das Problem, dass die Absaugleistung an die aktuellen Bedingungen der Operation angepasst werden müssen. Wie beschrieben, spielen hier die verwendeten Instrumente, aber auch die aktuellen Leckraten am Abdomen eine entscheidende Rolle.

Stand der Technik ist weiterhin, dass dem Operateur am Gerät verschiedene Stufen für die Absaugleistung angeboten werden (beispielsweise: Niedrig / Mittel / Hoch). Der Operateur hat damit die Verantwortung, die optimale Absaugstufe festzulegen. Sollte die Absaugleistung zu hoch eingestellt sein, kann der Insufflator den Bauchdruck nicht aufrecht erhalten. Dies bemerkt der Operateur üblicherweise erst, wenn der Druck bereits deutlich zurückgegangen ist. Weiterhin ist es für den Operateur schwierig, im Vorhinein zu erkennen, dass auch eine höhere Absaugleistung möglich und auch nötig ist. Möglicherweise wählt der Operateur dann erst gar nicht die höhere Absaugleistung aus, um den Druckabfall nicht zu riskieren.

Weiterer Stand der Technik wird gebildet durch die folgenden Dokumente:
- WO 2011/041387 A1 beschreibt ein Gaszuleitungssystem für laparoskopische Anwendungen. Hierbei kann Gas aus einem Behandlungsraum abgesaugt werden. Das Gas wird dem Körper anschließend wieder zugeführt, so dass keine Druckreduktion möglich ist.
- DE 4219859 A1 beschreibt einen Insufflator mit der Möglichkeit einer Gaszuleitung und einer Gasableitung. Gaszu- und -ableitung wird über eine einzige Leitung vorgenommen.
- WO 2004/009167 A1 beschreibt eine Vorrichtung zur Insufflation einer Körperhöhle einen Schlauch, welcher seinerseits eine Gasableitung enthält, die zu einer Messvorrichtung führt. Die Messvorrichtung kann insbesondere prüfen, ob sich im Abdomen einen zündfähiges Gasgemisch angesammelt hat.
- WO 2012/012379 A1 beschreibt ein Gasmanagementsystem für die Laparoskopie, bei dem Gasflüsse auf der Basis von In-vivo-Sensoren gesteuert werden.

Es besteht daher Bedarf an einer Vorrichtung für die minimal-invasive Chirurgie, insbesondere die Laparoskopie, die es erlaubt, die höchstmögliche Absaugrate für Rauchgase aus dem Behandlungsraum (insbesondere dem Abdomen) zu bestimmen und einzustellen, so dass kein siginifikanter Druckabfall im genannten Volumen stattfindet. Dabei sollen die im Markt üblichen Trokare verwendet werden können. Die Lösung des Problems wird durch die nachfolgend beschriebene erfindungsgemäße Vorrichtung gewährleistet.

### Lösung der Aufgabe

Die Lösung der Aufgabe erfolgt durch den Gegenstand der Patentansprüche.

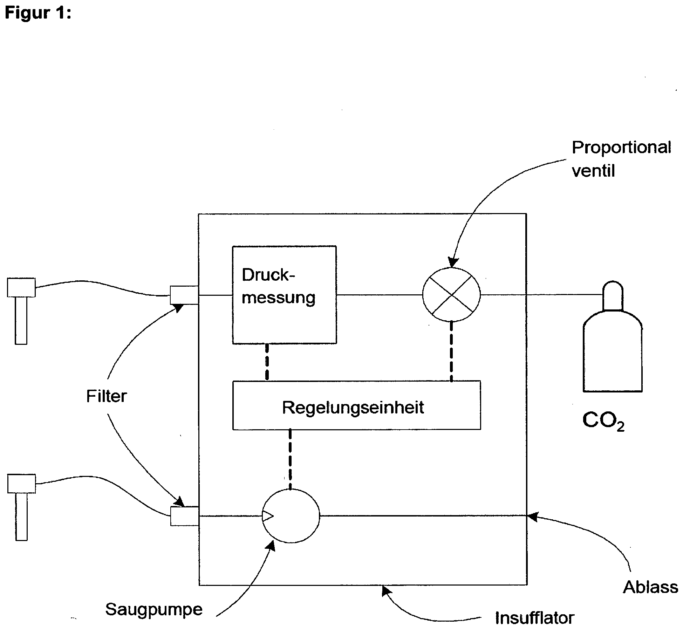

Hier beschrieben, aber nicht erfindungsgemäß, ist ein Insufflator mit integrierter Absaugpumpe. Eine derartige Pumpe ist beispielhaft in der Figur 1 dargestellt. Der Insufflator enthält zunächst einen üblichen Anschluss an eine Druckgasflasche mit einem Gas, welches für medizinische Zwecke geeignet ist, z. B. Kohlendioxid. Das Gas wird zunächst über ein Proportionalventil und einem sterilem Schlauch einem Trokar zugeführt. Innerhalb des Insufflators wird sowohl der Druck, als auch der Gasfluss (Flow) der Leitung gemessen. Optional wird noch ein Filter eingeführt, der Partikel zurückhält.

Ein zweiter Trokar wird an eine in den Insufflator integrierte Saugpumpe angeschlossen. Dabei wird optional wiederum ein Filter zwischengeschaltet, der Partikel, Tröpfchen und/oder giftige Gase absorbieren kann. Das abgepumpte Insufflationsgas kann dann in die Atmosphäre entlassen werden. Der Insufflator wird über eine Schalteinheit (Control Circuit) gesteuert. Dieser regelt insbesondere die Leistung der Saugpumpe auf elektronischem Wege.

Hier beschrieben ist ein Insufflator für die minimal-invasive Chirurgie, enthaltend
a) Gasanschluss mit Proportionalventil,
b) Zuführleitung mit Druckmessung, Filter und Anschluss an einen ersten Trokar,
c) zweiter Trokar mit Absaugschlauch und Filter, angeschlossen an eine Absaugvorrichtung mit regelbarer Absaugleistung,
d) elektronische Regelungseinheit,
wobei die elektronische Regelungseinheit die Absaugvorrichtung in Abhängigkeit vom Druck in der Zuführleitung regelt.

In einer hier beschriebenen Ausführungsform ist der Insufflator dadurch gekennzeichnet, dass die elektronische Regeleinheit durch Steuerung der Absaugvorrichtung die Absaugrate verkleinert, wenn der Druck in der Zuführleitung einen Schwellwert überschreitet und/oder dass die elektronische Regeleinheit durch Steuerung der Absaugvorrichtung die Absaugrate erhöht, wenn der Druck in der Zuführleitung einen Schwellwert unterschreitet.

Der Schwellwert zur Verringerung der Absaugrate für den Druck in der Zuführleitung kann beispielsweise zwischen 8,0kPa (60mm Hg) und 10,7 kPa (80 mmHg) liegen. Der Schwellwert zur Erhöhung der Absaugrate für den Druck in der Zuführleitung kann beispielsweise zwischen 0 und 6,7 kPa (50 mmHg) liegen. In speziellen Ausführungsformen sind die Schwellwerte am Insufflator einstellbar.

Die maximale Flussrate des Insufflators wird maßgeblich von den angeschlossenen Instrumenten am Insufflationsschlauch bestimmt. Bei Verwendung eines kleinen bzw. engen Trokars in Kombination mit einem großen Instrument kann die maximale Insufflationsleistung 4 - 6 l/min oder weniger betragen. Bei Verwendung von großen Trokaren kann die maximale Insufflationsleistung bei 15 - 20 l/min oder sogar darüber hinaus liegen.

Der Insufflator regelt das nachzufüllende Volumen nach dem jeweiligen Bedarf.

Selbstverständlich wird bereits aus Sicherheitsgründen ein maximaler Druck im Zuführschlauch definiert, der nicht überschritten werden darf. Dieser maximale Druck limitiert den maximalen Gasfluss.

Der Bedarf zum Nachfüllen von Kohlendioxid wird durch permanente sowie kurzzeitige Leckagen erzeugt. Die im Patienten eingesetzen Trokare weisen bauartbedingt minimale Leckagen auf. Weiterhin kann die aktive Rauchgasabsaugung gewissermaßen als eine permanente Leckage angesehen werden, die durch den Insufflator ausgeglichen werden muss.

Wenn beispielsweise intraoperativ eine Absaugung mit einer Flussrate von 4 l/min. vorhanden ist und eine systembedingte Leckage von 3 l/min., dann muss der Insufflator einen Flow von ∼ 7 l/min im Zufluss einstellen. Der Insufflator wird zum Erreichen des Zielflow den Druck im Zuführschlauch solange erhöhen, bis sich entweder der Flow einstellt, oder der maximal zulässige Druck erreicht wurde. Wird der maximale Druck erreicht bevor der Zielflow erreicht wurde, kann die Leckage durch den Insufflator nicht ausgeglichen werden. Sollte dieser Zustand länger anhalten, würde der abdominale Druck fallen. Aus diesem Grund muss der Insufflator in diesem Fall die Absaugleistung verringern.

Wenn jedoch ausreichend Kapazität im Schlauch bzw. Instrument vorhanden ist, kann der geforderte Flow von 7 l/min. eingestellt werden, ohne dass der maximale Druck erreicht wird. In diesem Fall kann die Absaugleistung sogar noch erhöht werden.

Der erreichte Druck im Zuführschlauch im Verhältnis zum maximalen Druck während der Insufflation ist also ein Maß, welches die noch verfügbare Kapazität im Schlauch bzw. Instrument anzeigt.

Die Regelung der Leistung kann mittels des oben beschriebenen Verhältnisses aus erreichtem Treiberdruck zu maximalem Treiberdruck geregelt werden. Dabei kann direkt die Eingangsspannung der Pumpe variiert werden.

Im Ergebnis wird die Leistung der Rauchgasabsaugung auf die aktuellen Bedingungen der Insufflationsleistung angepasst bzw. optimiert. Damit wird die maximale Absaugleistung eingestellt, ohne dass der Patientendruck sich ändern würde.

Der maximale Treiberdruck der erfindungsgemäßen Vorrichtung liegt üblicherweise bei 80 mmHg.

### Alternative Ausführungsformen

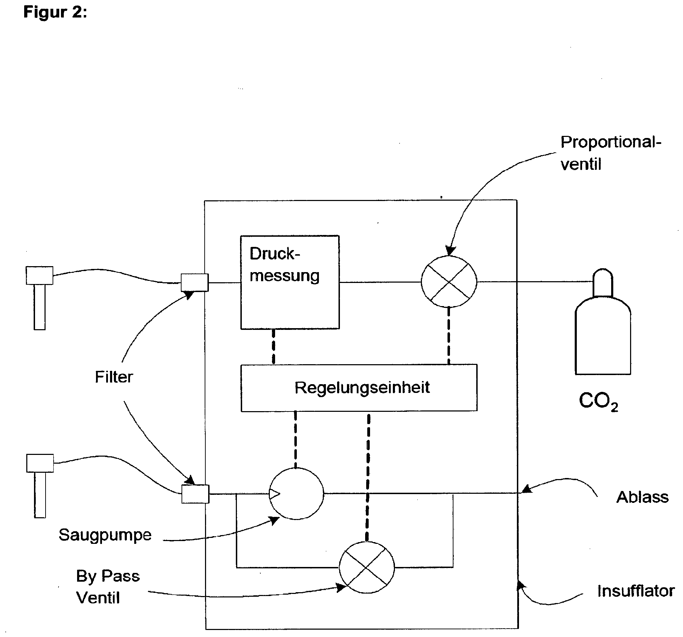

Der Fachmann auf dem Gebiet, angeregt von der vorliegenden Beschreibung, kann den hier beschriebenen Grundgedanken auch mit anderen Mitteln realisieren. So kann beispielsweise die Saugpumpe über einen ByPass-Ventil gesteuert werden (Figur 2). Hierzu kann beispielsweise die Pumpe auf eine bestimmte Leistung eingestellt werden, welche weitestgehend konstant ist, und die Regelung der Leistung erfolgt dann über das ByPass-Ventil.

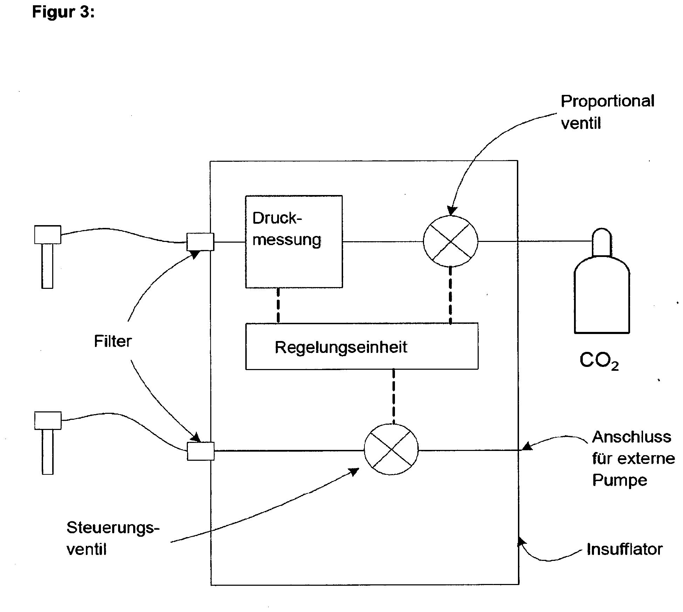

Erfindungsgemäß wird ein Steuerungsventil direkt in die Absaugleitung positioniert (Figur 3). Auf diese Weise wird erfindungsgemäß eine externe Pumpe verwendet, beispielsweise die im OP vorhandene Wandsaugung. Das Kontrollsystem des Insufflators regelt dann die Absaugleistung über das dargestellte Steuerungsventil. Dabei ist die elektronische Regeleinheit zur Steuerung der von der externen Pumpe verursachten Absaugleistung erfindungsgemäß so konfiguriert, dass die Absaugrate verkleinert wird, wenn der Druck in der Gaszuführleitung einen Schwellwert überschreitet, der zwischen 8,0kPa (60mm Hg) und 10,7 kPa (80 mmHg) liegt, und dass die Absaugrate erhöht wird, wenn der Druck in der Gaszuführleitung einen Schwellwert unterschreitet, der zwischen 0 und 6,7 kPa (50 mmHg) liegt.

In weiteren Ausführungsformen der Erfindung kann die Gaszuführleitung vom Insufflator zum Patienten auch heizbar ausgestaltet sein, beispielsweise über einen Schlauch, wie er in der WO2014/111084 beschrieben wird. Alternativ kann die Gaszuführleitung vom Insufflator zum Patienten nicht nur heizbar sondern auch zur Befeuchtung des Gases eingerichtet sein, beispielsweise über einen Schlauch, wie er in der WO2014/111083 beschrieben wird.

## Patentansprüche

1. Insufflator für die minimal-invasive Chirurgie, enthaltend
a) Gasanschluss mit Proportionalventil,
b) einen ersten Trokar,
c) eine Gaszuführleitung für Gas aus dem Gasanschluss mit Druckmessung, Filter und Anschluss an den ersten Trokar,
d) eine externe Pumpe,
e) einen zweiten Trokar mit Absaugschlauch und Filter, über eine Absaugleitung des Insufflators angeschlossen an die externe Pumpe
und
f) eine elektronische Regelungseinheit,
wobei ein Steuerungsventil in der Absaugleitung positioniert ist,
wobei die elektronische Regelungseinheit konfiguriert ist, mittels des Steuerungsventils die von der externen Pumpe verursachte Absaugleistung in Abhängigkeit vom Druck in der Gaszuführleitung zu regeln, und
wobei die elektronische Regeleinheit zur Steuerung der Absaugleistung so konfiguriert ist,
dass die Absaugrate verkleinert wird, wenn der Druck in der Gaszuführleitung einen Schwellwert überschreitet, der zwischen 8,0kPa (60mm Hg) und 10,7 kPa (80 mmHg) liegt,
und
dass die Absaugrate erhöht wird, wenn der Druck in der Gaszuführleitung einen Schwellwert unterschreitet, der zwischen 0 und 6,7 kPa (50 mmHg) liegt.

2. Insufflator gemäß mindestens einem der Ansprüche 1, **gekennzeichnet durch** eine zwischen 0 und 50l/min regelbare Insufflationsleistung.

3. Insufflator gemäß Anspruch 2, **gekennzeichnet durch** eine zwischen 0 und 20 l/min regelbare Absaugleistung.

4. Insufflator gemäß Anspruch 3, **gekennzeichnet durch** eine zwischen 0 und 10 l/min regelbare Absaugleistung.

5. Insufflator gemäß mindestens einem der Ansprüche 1-4, **gekennzeichnet durch** eine Gasheizvorrichtung in der Gaszuführleitung.

6. Insufflator gemäß mindestens einem der Ansprüche 1-4, **gekennzeichnet durch** eine Gasheizvorrichtung in der Gaszuführleitung, und wobei die Gaszuführleitung zur Gasbefeuchtung eingerichtet ist.

## Claims

1. An insufflator for the minimally invasive surgery, comprising
a) a gas port with a proportional valve,
b) a first trocar,
c) a gas supply line for gas from the gas port with pressure measurement, filter, and connection to the first trocar,
d) an external pump,
e) a second trocar with suction tube and filter, connected to the external pump via a suction line of the insufflator, and
f) an electronic regulation unit,
wherein a control valve is positioned in the suction line,
wherein the electronic regulation unit is configured to regulate, by means of the control valve, the suction power induced by the external pump depending on the pressure in the gas supply line, and
wherein the electronic regulation unit is configured for controlling the suction power such that the suction rate is reduced, when the pressure in the gas supply line exceeds a limit value between 8.0 kPa (60 mm Hg) and 10.7 kPa (80 mm Hg), and that the suction rate is increased, when the pressure in the gas supply line goes below a limit value between 0 and 6.7 kPa (50 mm Hg).

2. The insufflator according to claim 1, **characterized by** an insufflation power controllable between 0 and 50 1/min.

3. The insufflator according to claim 2, **characterized by** a suction power controllable between 0 and 20 l/min.

4. The insufflator according to claim 3, **characterized by** a suction power controllable between 0 and 10 l/min.

5. The insufflator according to at least one of claims 1 to 4, **characterized by** a gas heating device in the gas supply line.

6. The insufflator according to at least one of claims 1 to 4, **characterized by** a gas heating device in the gas supply line and wherein the gas supply line is configured for gas humidification.

## Revendications

1. Insufflateur pour la chirurgie mini-invasive, comprenant
a) un raccordement au gaz avec une vanne proportionnelle,
b) un premier trocar,
c) une conduite d'alimentation de gaz pour gaz à partir du raccordement au gaz avec mesurage de pression, filtre, et raccordement au premier trocar,
d) une pompe externe,
e) un deuxième trocar avec tuyau d'aspiration et filtre, raccordé à la pompe externe par l'intermédiaire d'une conduite d'aspiration de l'insufflateur, et
f) une unité de régulation électronique,
dans lequel une vanne de commande est positionnée dans la conduite d'aspiration,
dans lequel l'unité de régulation électronique est configurée à réguler, au moyen de la vanne de commande, la puissance d'aspiration induite par la pompe externe en fonction de la pression dans la conduite d'alimentation de gaz, et
dans lequel l'unité de régulation électronique est configurée pour commander la puissance d'aspiration de façon que le débit d'aspiration soit réduit, si la pression dans la conduite d'alimentation de gaz passe au-dessus d'une valeur seuil entre 8,0 kPa (60 mm Hg) et 10,7 kPa (80 mm Hg), et que le débit d'aspiration soit augmenté, si la pression dans la conduite d'alimentation de gaz passe au-dessous d'une valeur seuil entre 0 et 6,7 kPa (50 mm Hg).

2. Insufflateur selon la revendication 1, **caractérisé par** une puissance d'insufflation commandable entre 0 et 50 l/min.

3. Insufflateur selon la revendication 2, **caractérisé par** une puissance d'aspiration commandable entre 0 et 20 l/min.

4. Insufflateur selon la revendication 3, **caractérisé par** une puissance d'aspiration commandable entre 0 et 10 1/min.

5. Insufflateur selon au moins une des revendications 1 à 4, **caractérisé par** un dispositif de chauffage de gaz dans la conduite d'alimentation de gaz.

6. Insufflateur selon au moins une des revendications 1 à 4, **caractérisé par** un dispositif de chauffage de gaz dans la conduite d'alimentation de gaz, et dans lequel la conduite d'alimentation de gaz est configurée pour l'humidification de gaz.
